# FASCICULE DE BREVET EUROPEEN

(11) **EP 3 066 066 B1**
(45) Date de publication et mention de la délivrance du brevet: **25.04.2018**
(21) Numéro de dépôt: 14806022.1
(22) Date de dépôt: 06.11.2014
(51) Int. Cl.: C07C 67/02, C07C 69/58, C07C 67/40, C07F 15/00

(54) **PROCÉDÉ DE SYNTHÈSE D'ESTERS**
VERFAHREN ZUR SYNTHESE VON ESTERN
METHOD FOR SYNTHESISING ESTERS

(30) Priorité: 08.11.2013 FR 1360982
(43) Date de publication de la demande: 14.09.2016
(73) Titulaire: Pivert, 60280 Venette (FR); Centre National de la Recherche Scientifique (C.N.R.S.), 75794 Paris Cedex 16 (FR); Université des Sciences et Technologies de Lille 1, 59655 Villeneuve-d'Ascq Cedex (FR); Ecole Centrale De Lille, 59651 Villeneuve d'Ascq Cedex (FR)
(72) Inventeur: DUMEIGNIL, Franck, F-59273 Fretin (FR); DESSET, Simon, F-59000 Lille (FR); PAUL, Sébastien, F-59158 Thun Saint Amand (FR); RAFFA, Guillaume, F-69610 Les Halles (FR); ZHANG, Lei, F-59650 Villeneuve d'Ascq (FR)
(74) Mandataire: McDade, Sophie V.G.A.
(86) Numéro de dépôt international: PCT/FR2014/052840
(87) Numéro de publication internationale: WO 2015/067900

(56) Documents cités:
- WO-A1-2013/171302
- US-A1- 2011 237 814
- A. PICCIRILLI ET AL: "Hydrogénation sélective de l'oléate de méthyle en alcohol oléique en présence de catalyseurs ruthénium-étain supportés", BULLETIN DE LA SOCIETE CHIMIQUE DE FRANCE., vol. 132, 1995, pages 1109-1117, XP008171725, SOCIETE FRANCAISE DE CHIMIE. PARIS. ISSN: 0037-8968
- BOOPATHY GNANAPRAKASAM ET AL: "Ruthenium pincer-catalyzed acylation of alcohols using esters with liberation of hydrogen under neutral conditions", ADVANCED SYNTHESIS AND CATALYSIS, vol. 352, 2010, pages 3169-3173, XP002729156, WILEY, WEINHEIM ISSN: 1615-4169
- DENIS SPASYUK ET AL: "From Esters to Alcohols and Back with Ruthenium and Osmium Catalysts", ANGEWANDTE CHEMIE INTERNATIONAL EDITION, vol. 51, no. 11, 12 March 2012 (2012-03-12), pages 2772-2775, XP55129680, ISSN: 1433-7851, DOI: 10.1002/anie.201108956

## Description

### Domaine technique

L'invention porte sur un procédé de synthèse d'esters à partir de produits de départ biosourcés par couplage déshydrogénant en présence d'un catalyseur. Les produits de départ peuvent être des esters biosourcés tels que des esters méthyliques d'acides gras (FAME) issus d'oléagineux, par exemple l'oléate de méthyle.

### Art antérieur

De nombreux esters, et notamment l'acétate d'éthyle, sont synthétisés à l'échelle industrielle en utilisant des produits de départ d'origine fossile (l'éthylène dans le cas de l'acétate d'éthyle) via des procédés multi-étapes. Le marché mondial de l'acétate d'éthyle était de 2,5 million de tonnes/an en 2008.

Il est connu d'utiliser un catalyseur à base de ruthénium pour réaliser le couplage déshydrogénant de l'éthanol en utilisant par exemple le carbonylchlorohydrido [bis(2-diphenylphosphinoethyl)amino]ruthenium(II), de formule **A**, (CAS : 1295649-40-9), Trade Name : Ru-MACHO, ou D (voir ci-dessous) (cf. M. Nielsen, H. Junge, A. Kammer and M.Beller, Angew. Chem. Int. Ed., 2012, 51, 5711-5713 et EP2599544A1) ou le *trans*-RuCl2(PPh3)[PyCH2NH(CH2)2PPh2], de formule **B**, (cf. D. Spasyuk and D. Gusev, Organometallics, 2012, 31, 5239-5242). Ces catalyseurs **A** et **B** requièrent cependant la présence de tBuOK ou EtONa, pour être actifs. Le catalyseur **D** requière un long temps de réaction et une charge catalytique élevée pour obtenir des rendements au maximum de 90% en ester.

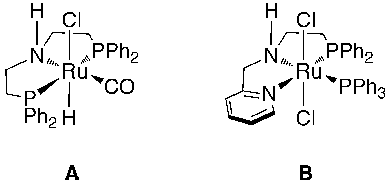

Un autre catalyseur utilisé pour cette même réaction est le catalyseur carbonylhydrido(tetrahydroborato)[bis(2-diphenylphosphinoethyl)amino]ruthenium(II), de formule **C** (CAS : 1295649-41-0), Trade Name : Ru-MACHO-BH. Cette réaction est décrite dans la demande de brevet WO2012/144650 où cette synthèse requière la présence d'un accepteur d'hydrogène tel une cétone, par exemple, la 3-pentanone. En plus de dissoudre les différentes espèces (catalyseur et substrat) la 3-pentanone joue le rôle d'accepteur d'hydrogène. Ainsi une quantité au moins stoechiométrique de 3-pentanone est utilisée dans les exemples et les réactions décrites ne s'accompagnent donc pas du dégagement d'hydrogène gazeux.

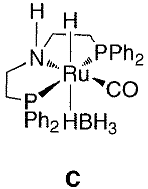

Il est également connu d'utiliser un catalyseur à base de ruthénium pour réaliser le couplage déshydrogénant du butanol en utilisant par exemple trans-RuH₂(CO)[HN(C₂H₄P*i*Pr₂)₂], de formule **D**, (M. Bertoli, A. Choualeb, A. J. Lough, B. Moore, D. Spasyuk and D. Gusev, Organometallics, 2011, 30, 3479-3482) ou le [RuH (PNN-)(CO)], de formule **E**, (cf. J. Zhang, G. Leitus, Y. Ben-David and D. Milstein, J. Am. Chem. Soc., 2005, 127,10840-10841) en présence de solvant et en l'absence de base et d'accepteur d'hydrogène. Cependant ces catalyseurs nécessitent de longs temps de réaction et des charges catalytiques élevées pour obtenir des rendements au maximum de 90% en ester.

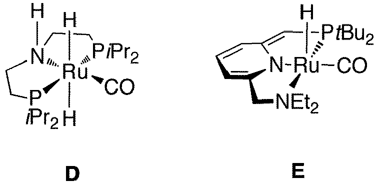

La mise en place de tels procédés à l'échelle industrielle présente cependant de nombreux désavantages. Un de ces désavantages est la nécessité d'effectuer plusieurs étapes de purification pour isoler les produits de la réaction ce qui complexifie notablement le procédé. Un autre problème est l'utilisation de produits organiques, tels que des solvants, en sus des produits de départs. L'utilisation de tels produits augmente sensiblement l'impact environnemental de telles synthèses, ce qui est bien sûr à éviter. Il a maintenant été réalisé un procédé qui résout ces problèmes et qui offre ainsi une alternative réaliste aux procédés industriels de synthèse d'esters utilisant des ressources fossiles. Ce procédé permet en effet d'associer de hauts rendements à des étapes de synthèse et de purification simplifiées au niveau de la mise en oeuvre.

L'art antérieur est décrit dans le document D1 A. PICCIRILLI ET AL: " Hydrogénation sélective de l'oléate de méthyle en alcohol oléique en présence de catalyseurs ruthénium-étain ", BULLETIN DE LA SOCIETE CHIMIQUE DE FRANCE., vol. 132, 1995, pages 1109-1117, SOCIETE FRANCAISE DE CHIMIE. PARIS. ISSN: 0037-8968.

Le document D1 décrit un procédé pour la préparation d'un ester comportant deux chaînes identiques à partir d'un ester de méthyle utilisant catalyseur de ruthénium et d'autres métaux.

On peut également citer comme faisant parti de l'art antérieur le document D2 US 2011/237814 A1 (KURIYAMA WATARU [JP] ET AL) 29 septembre 2011 (201 10929) qui décrit une méthode pour produire des alcohols par hydrogénation-reduction de kétones, esters ou lactones utilisant un nouveau mélange de ruthénium-carbonyle comme catalyseur.

### Description de l'invention

Selon un mode de réalisation l'invention porte sur un procédé de synthèse d'un deuxième ester à partir d'un premier ester, procédé comprenant les étapes suivantes:
a) la mise en présence d'un premier ester et d'un catalyseur de formule 1 et de dihydrogène pour obtenir un premier alcool et un second alcool;
b) Séparer ledit second alcool du milieu réactionnel;
c) faire réagir, par exemple par chauffage, ledit premier alcool avec ledit catalyseur de formule 1 pour obtenir un deuxième ester et du dihydrogène,et
d) recycler le dihydrogène obtenu à l'étape c) en l'introduisant à l'étape a) ; ladite formule 1 étant : 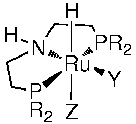
   où R sont des groupements, identiques ou différents, choisis dans le groupe constitué par le cyclohexyle, le phényle, l'isopropyle et l'éthyle;
   où Y est une phosphine PR'3, un radical CO ou un atome d'hydrogène, R', identiques ou différents, étant des groupements alkyles en C1-C12 ou aryle en C6-C12; et
   où Z est un atome d'hydrogène, un groupement HBH3 ou un atome d'halogène.

Selon un mode de réalisation préféré de l'invention, l'étape c) s'effectue sans ajout d'un accepteur d'hydrogène. De manière avantageuse les étapes a) à c) s'effectuent sans ajout additionnel d'un accepteur d'hydrogène.

Selon un mode de réalisation préféré de l'invention, l'étape c) s'effectue sans ajout de solvant. De manière avantageuse les étapes a) à c) s'effectuent sans ajout de solvant.

Selon un mode de réalisation préféré de l'invention, l'étape c) s'effectue sans ajout de base. De manière avantageuse les étapes a) à c) s'effectuent sans ajout de base.

Selon un mode de réalisation préféré de l'invention, l'étape c) s'effectue à la fois sans ajout de solvant, sans ajout d'un accepteur d'hydrogène, et sans ajout de base.

L'expression « accepteur d'hydrogène » désigne les composés organiques qui sont susceptibles de réagir avec de l'hydrogène moléculaire, H₂, pour former un nouveau composé dans les conditions réactionnelles de la synthèse d'ester. En particulier il peut s'agir de composés tels que les cétones, les aldéhydes et les alcènes.

L'absence de composés pouvant réagir avec l'hydrogène moléculaire pour l'absorber à l'étape c) est particulièrement avantageux car il permet notamment la production d'hydrogène gazeux H₂ qui est facilement isolé du milieu réactionnel et qui peut donc être utilisé subséquemment, par exemple pour l'étape a). De plus, cela évite la formation stoechiométrique du produit d'hydrogénation de l'accepteur d'hydrogène, facilitant les étapes de purification en aval.

Ainsi, le procédé selon l'invention permet également d'obtenir de l'hydrogène moléculaire gazeux. Celui-ci se sépare du milieu réactionnel par simple séparation de phase et peut être évacué et/ou recueilli directement puis réutilisé dans l'étape a) du procédé. Ce procédé est donc particulièrement adapté à un dispositif de production industriel car il permet un recyclage simple et efficace des sous-produits de la réaction, limitant ainsi le besoin en hydrogène additionnel pour la première étape.

Le terme de « base » désigne un composé capable de capter un ou plusieurs proton(s). Dans le cadre de l'invention le terme de base désigne tout particulièrement des bases telles la soude, ou des sels de métaux alcalins alkoxylés comme EtONa, MeONa ou ^{t}BuOK.

De manière préférée, la réaction est réalisée en l'absence de toluène ou de xylène et en particulier de solvant.

Le terme solvant désigne une substance, liquide à sa température d'utilisation, qui a la propriété de dissoudre, de diluer ou d'extraire les alcools et éventuellement le catalyseur sans les modifier chimiquement dans les conditions réactionnelles de la synthèse d'esters. Eventuellement un solvant ne se modifie pas lui-même dans les conditions de la réaction à laquelle il participe. Il peut s'agir de composés tels que l'eau, les solvants inorganiques, et les solvants organiques de type hydrocarboné, oxygéné, et halogéné.

Ce solvant en l'absence duquel la réaction est réalisée peut évidemment être également un accepteur d'hydrogène et/ou une base. Ainsi, dans le cadre de l'invention le terme solvant désigne notamment les cétones, telles la 3-pentanone, l'acétone ou la cyclohexanone. Il désigne également les composés hydrocarbonés aromatiques ou aliphatiques, éventuellement halogénés, les éthers et les alcools.

L'expression « absence de » est utilisée dans son acception normale qui implique l'absence dans le mélange réactionnel initial d'une quantité suffisante du composé pour jouer un rôle effectif dans la réaction ainsi que l'absence d'ajout extérieur de ce composé lors de la réaction. Par exemple la présence dans le milieu réactionnel d'une quantité minime (par exemple sous forme de trace) d'un accepteur d'hydrogène, d'une base ou d'un solvant n'influencera pas de manière sensible la réaction. Ainsi l'absence de solvant implique l'absence d'une quantité suffisante pour dissoudre/diluer/extraire le ou les produits de départs (c.à.d. le ou les esters et le ou les alcool(s)) ainsi que le catalyseur. Pour un solvant cette quantité est généralement supérieure aux nombres de moles de réactifs, c'est-à-dire que le solvant est généralement présent dans le milieu réactionnel dans une concentration molaire supérieure ou égale à 50%.

De préférence, l'expression « absence de» implique une concentration molaire dudit composé inférieure à 10%, et plus particulièrement inférieure à 5%, voire son absence totale c'est-à-dire inférieure à 0,001%.

De préférence, dans la formule 1 du catalyseur, Z n'est pas un atome d'halogène.

De préférence, le groupement R' de la formule 1 est un groupement méthyle, éthyle, isopropyle ou phényle. Il est également préféré que les groupements R' soient identiques.

Avantageusement, les quatre groupements R sont identiques.

De préférence R est un groupement cyclohexyle ou isopropyle.

Selon un mode de réalisation particulier de l'invention le premier ester, où ester de départ, présente la formule suivante : 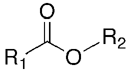 où R₁ est un groupement alkyle, linéaire ou ramifié, de C₂ à C₃₂, de préférence de C₂ à C₂₂, et R₂ est un groupement alkyle de C₁ à C₆. R₁ et R₂ peuvent également comporter des insaturations ou des fonctionnalisations telles que des groupes hydroxyle, amines, et cétones:
L'ester de départ, ou premier ester, peut également présenter la formule suivante : 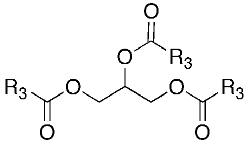 où R₃ sont des groupements alkyles de C₂ à C₃₂, de préférence de C₈ à C₂₂, identiques ou différents, linéaire ou ramifiés. R₃ peut également comporter des insaturations ou des fonctionnalisations telles que, par exemple, au moins un groupe hydroxyle, amine ou cétone.

Par exemple, le premier ester peut être un ester méthylique d'acide gras (FAME) tel que l'oléate de méthyle.

De préférence, la charge de catalyseur utilisée dans le procédé selon l'invention, et plus particulièrement aux étapes a) et c), est inférieure à 10000 ppm, plus particulièrement inférieure à 1000 ppm encore plus particulièrement inférieure à 500 ppm. Cette charge peut-être par exemple d'environ 50 ± 10 ppm.

De préférence, la charge de catalyseur utilisée dans le procédé selon l'invention est choisie dans une gamme allant de 10000 ppm à 1 ppm, plus particulièrement de 1000 ppm à 10 ppm et encore plus particulièrement de 500 ppm à 50 ppm. Cette charge peut-être par exemple d'environ 225 ± 10 ppm. Avantageusement la charge est choisie dans une gamme allant de 500 ppm à 1 ppm, avantageusement de 200 ppm à 20 ppm, plus avantageusement de 60 ppm à 40 ppm.

Les proportions ci-dessus sont données par rapport à la masse totale des produits de départ.

De préférence l'étape a) est réalisée sous pression d'hydrogène gazeux, ladite pression d'hydrogène pouvant être choisie dans la gamme de 10 à 50 bars, de préférence de 20 à 30 bars, par exemple à une pression d'environ 20.

L'étape a) est réalisée à une température allant de 5°C à 150°C, avantageusement de 75°C à 125°C, plus avantageusement à 100°C.

De manière avantageuse, l'étape d'isolation b) est effectuée par des techniques connues de séparation telle que l'évaporation sous vide ou l'extraction aqueuse. Le produit de la réaction comprenant généralement un second alcool, non désiré, celui-ci est avantageusement extrait du milieu réactionnel. Ainsi la réaction d'estérification subséquente permet de coupler 2 molécules du même alcool pour créer un nouvel ester, différent de celui de départ.

De préférence l'étape c) comprend le chauffage du milieu réactionnel et est réalisée à une température choisie dans une gamme de température allant de 200°C à 15°C, plus particulièrement de 150°C à 40°C et encore plus particulièrement de 130°C à 80°C. Cette température peut-être d'environ 130±1°C. De préférence la température peut aller de 5 à 200 °C, avantageusement de 100 à 150°C, plus avantageusement à environ 130°C.

De préférence la réaction de l'étape c) est réalisée à une pression allant de 20 bars à 1 bar. Avantageusement aucune pression particulière n'est appliquée et la réaction est réalisée à la pression atmosphérique ou dans un système ouvert.

Il est avantageux que la pression permette un dégagement d'hydrogène. Ainsi une pression atmosphérique, ou une pression inférieure à la pression atmosphérique (dite sous vide).

De préférence, le catalyseur utilisé dans les étapes a) et c) du procédé est de préférence le même.

Dans le procédé selon l'invention, le composé de départ (ester) peut être présent seul ou en mélange avec d'autres réactifs comme d'autres esters.

De plus le composé de départ peut être utilisé sous forme purifiée ou sous forme brute, notamment non raffinée, ceci en particulier lorsque le composé est obtenu à partir d'huiles végétales (par ex. d'oléagineux).

Selon un aspect particulier de l'invention, l'étape c) du procédé, et de préférence toutes les étapes a) à c) est réalisé en l'absence de tout additif (autre que le catalyseur) pouvant avoir un effet sur la réaction de couplage de l'alcool et/ou sur la production d'hydrogène moléculaire.

Selon un autre aspect préféré de l'invention, le procédé ne comprend pas d'étape de séchage et/ou de dégazage produits de départ ou du milieu réactionnel. En effet il a été déterminé de manière surprenante que le catalyseur (et particulièrement les catalyseurs où Z est HBH₃ tel que le Ru-MACHO-BH, de formule **C** ou 1a)) reste actif en présence d'air et de traces d'eau.

Il apparait en effet que, de manière inattendue, ces conditions réactionnelles spécifiques notamment sans ajout de solvant, sans ajout d'accepteur d'hydrogène, sans ajout de base, en présence d'air et de traces d'eau, permettent d'obtenir à la fois un temps de réaction plus court et l'utilisation d'une charge très réduite de catalyseur tout en préservant un rendement élevé. Le fait de pouvoir s'affranchir de l'utilisation de composés chimiques additionnels tels que ceux mentionnés ci-dessus permet de réduire les coûts de fabrication, d'éviter les problèmes de corrosion liés à leur utilisation et donc de réduire de manière drastique l'impact environnemental de tels procédés. Cela permet également de simplifier grandement les opérations de séparation et/ou de purification des produits de la réaction en aval du procédé, opérations telles que l'élimination du solvant de la réaction par distillation, la neutralisation du milieu réactionnel utilisant une base, ou la séparation des produits de la réaction plus nombreux lors de l'emploi d'un accepteur d'hydrogène.

Selon un aspect particulier de l'invention, le catalyseur est de préférence un catalyseur de formule 1, dans laquelle Z est l'HBH₃ et/ou Y est un radical CO et R sont des radicaux phényles.

Selon un aspect de l'invention, le catalyseur est de préférence un catalyseur de formule 1 dans laquelle les quatre radicaux R sont identiques.

Selon un aspect particulièrement préféré de l'invention, le catalyseur utilisé est un catalyseur de formule 1 dans laquelle le groupement Z est H et le groupement Y est une phosphine PR'₃ où R' un groupement alkyle en C₁- C₁₂ ou aryle en C₆- C₁₂, en particulier un groupement méthyle, éthyle, isopropyle ou phényle.

Selon une variante préférée de l'invention, le catalyseur est le catalyseur de Formule 1b (R = *i*-Pr, Z = HBH₃, Y = CO).

Selon une variante préférée de l'invention, le catalyseur est le catalyseur de Formule 1a ou C (R = Ph, Z = HBH₃, Y = CO).

Selon une variante préférée de l'invention, le catalyseur est le catalyseur de Formule 1c (R = Cy, Z = HBH₃, Y = CO).

Selon une variante préférée de l'invention, le catalyseur est le catalyseur de Formule 6a (R = Ph, Z = H, Y = CO).

Selon une variante préférée de l'invention, le catalyseur est le catalyseur de Formule 6b (R = isopropyle, Z = H, Y = CO).

Selon une variante préférée de l'invention le catalyseur est le catalyseur de Formule 6c (R = Cy, Z = H, Y = CO).

L'invention porte également sur les catalyseurs décrits dans cette demande en tant que tels ainsi que sur leurs procédés de fabrication. En particulier un complexe de formule 1c, 1b, 6a et 6c, ainsi qu'un complexe de même formule où le substituant carbonyle est substitué par une phosphine est un objet de l'invention. Leurs utilisations dans des procédés de synthèse catalytique ainsi que de tels procédés sont également est également des objets de l'invention.

L'invention porte également sur un ester obtenu directement par le procédé décrit ci-dessus.

Selon un autre mode de réalisation de l'invention le procédé est un procédé de synthèse de cérides.

Selon un autre mode de réalisation il est également envisageable d'utiliser à l'étape a) et c) des catalyseurs de formules 1 différents. Il est de même envisageable d'utiliser l'alcool extrait à l'étape b) en tant que produit de départ pour la synthèse du second ester.

### Description détaillée de l'invention

### Exemple 1a : synthèse d'oléyle oléate (un céride) à partir d'un ester de méthyl utilisant le catalyseur 1a :

Le schéma réactionnel de la réaction est le suivant :

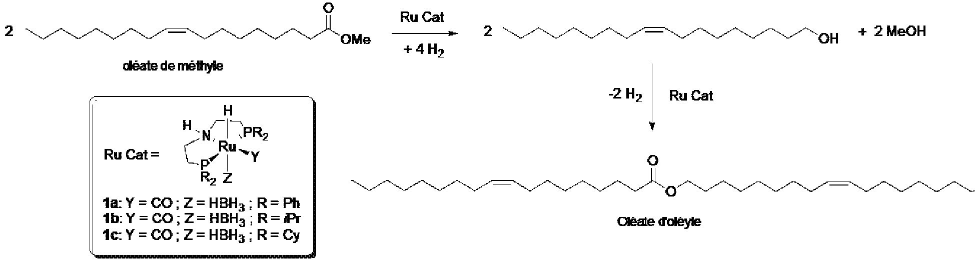

Le catalyseur 1a (4,5 mg ; 7,7 µmol) est introduit dans un autoclave contenant un barreau d'agitation. 2,3 g d'oléate de méthyle (7,7 mmol) est introduit via une seringue sous atmosphère d'argon. L'autoclave est ensuite purgé trois fois par un cycle vide/hydrogène puis environ 20 bars d'hydrogène sont introduits. Le système est chauffé à 100 °C à l'aide d'un bain d'huile et est agité magnétiquement pendant 18 heures. On obtient du méthanol ainsi que de l'alcool oléique. On note que les insaturations de la chaine grasse ne sont pas hydrogénées au cours de ce processus.

### Exemple 1b : synthèse d'oléyle oléate (un céride) à partir d'un ester de méthyl utilisant le catalyseur 1b :

Le schéma réactionnel de la réaction est le suivant :

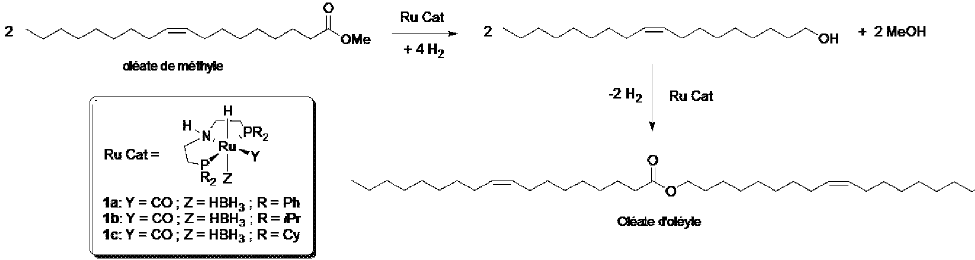

Le catalyseur 1b (3,4 mg; 7,7 µmol) est introduit dans un autoclave contenant un barreau d'agitation. 2,3 g d'oléate de méthyle (7,7 mmol) est introduit via une seringue sous atmosphère d'argon. L'autoclave est ensuite purgé trois fois par un cycle vide/hydrogène puis environ 20 bars d'hydrogène sont introduits. Le système est chauffé à 100 °C à l'aide d'un bain d'huile et est agité magnétiquement pendant 18 heures. On obtient du méthanol ainsi que de l'alcool oléique. On note que les insaturations de la chaine grasse ne sont pas hydrogénées au cours de ce processus.

### Exemple 1c : synthèse d'oléyle oléate (un céride) à partir d'un ester de méthyl utilisant le catalyseur 1c :

Le schéma réactionnel de la réaction est le suivant :

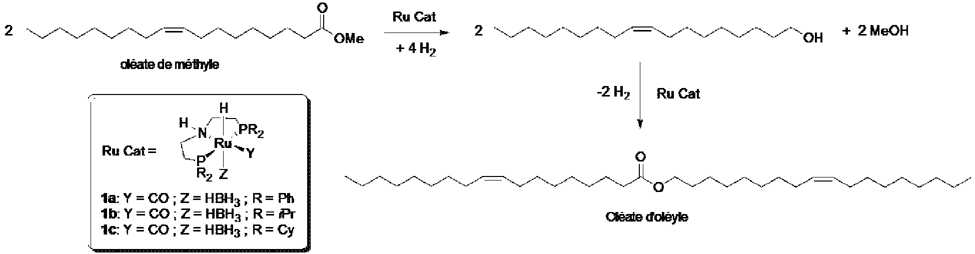

Le catalyseur 1c (4,7 mg; 7,7 µmol) est introduit dans un autoclave contenant un barreau d'agitation. 2,3 g d'oléate de méthyle (7,7 mmol) est introduit via une seringue sous atmosphère d'argon. L'autoclave est ensuite purgé trois fois par un cycle vide/hydrogène puis environ 20 bars d'hydrogène sont introduits. Le système est chauffé à 100 °C à l'aide d'un bain d'huile et est agité magnétiquement pendant 18 heures. On obtient du méthanol ainsi que de l'alcool oléique. On note que les insaturations de la chaine grasse ne sont pas hydrogénées au cours de ce processus.

Les sélectivités et les taux de conversion obtenus dans les exemples 1a, 1b, et 1c sont compilés dans le Tableau I :

**Tableau I**

| **Cat. (0,1 mol%)** | **Sélectivité Alcool oléique^{a}** | **Sélectivité Oléate d'oléyle^{a}** | **Conversion^{b}** |
|---|---|---|---|
| 1a | **80%** | **20%** | **93%** |
| 1b | **76%** | **24%** | **92%** |
| 1c | **73%** | **27%** | **93%** |
| ^{a}Sélectivité et conversion déterminée par RMN ¹H. ^{b}Conversion du méthyle oléate. | | | |

Le milieu réactionnel est transféré sous d'argon à l'aide d'une seringue dans un tube de Schlenk. Le méthanol est évaporé sous vide (20°C, 30 min, 1.10⁻³ mbar). Le tube de Schlenk est ensuite équipé d'un condenseur surmonté d'un bulleur. Le système contenant toujours le catalyseur 1a, 1b ou 1c dissous est ensuite chauffé à 130°C pendant 18h. Le dégagement concomitant d'hydrogène moléculaire gazeux est observé et peut donc être employé à la synthèse de l'alcool gras.

Les produits obtenus sont déterminés par RMN ((Spectromètre RMN Bruker Avance 1, 300 MHz, sonde 5 mm) et sont compilés dans le tableau II :

**Tableau II**

| **Cat. (0,1 mol%)** | **Sélectivité oléate d'oléyle** | **Sélectivité oléate de méthyle** | **Conversion** |
|---|---|---|---|
| **1a** | 95% | 5% | 58% |
| **1b** | 93% | 7% | 87% |
| **1c** | 95% | 5% | 86% |
| ^{a}Sélectivité et conversion déterminée par RMN ¹H. ^{b}Conversion de l'alcool oléique. | | | |

Cette transformation permet d'obtenir directement des cérides à partir des esters méthyliques issus de la trans-estérification des triglycérides.

Cette méthodologie est généralisable à d'autres méthyl esters d'acide gras (FAMEs) quelle que soit la longueur de la chaîne carbonée des esters méthyliques de départ.

### Exemple 2 : synthèse de cérides à partir d'un triglycéride :

### Exemple 2a : synthèse de butyrate de butyl à partir de tributyrate de glyceryl (tributyrine) en présence d'un solvant :

La synthèse de butyrate de butyl a été effectuée en deux étapes en utilisant le même catalyseur du ruthénium : hydrogénation de triglycéride suivie d'une déshydrogénation de l'alcool correspondant. Le schéma réactionnel de la réaction est le suivant :

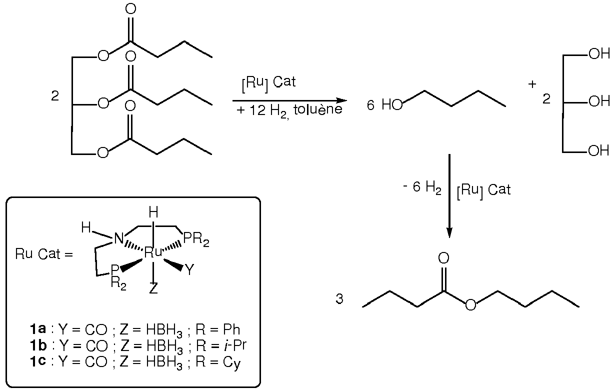

Etape 1 : Dans la boite à gant, 1 mol% du catalyseur 1a (30 mg ; 51.3 µmol), 0.516 g de tributyrate de glyceryl (1,7 mmol) et toluène (7 mL) sont successivement chargés sous atmosphère d'argon dans un autoclave contenant un barreau d'agitation. L'autoclave est ensuite purgé trois fois avec de l'hydrogène puis environ 40 bars d'hydrogène sont introduits. Le système est chauffé à 100 °C à l'aide d'un cordon chauffant électrique et est agité magnétiquement pendant 18 heures. Le mélange réactionnel a été filtré sur la célite si nécessaire avant d'être analysé par la RMN du proton et la chromatographie en phase gazeuse. Le taux de conversion est de l'ordre 96%. Les rendements pour le n-butanol et le butyrate de butyl sont respectivement 84 et 2%. De plus, la formation des mono et diester glycérides sont également observés avec les pourcentages estimés de l'ordre de 3%.

Etape 2 : Dans la boite à gant, le milieu réactionnel est transféré sous atmosphère d'argon à l'aide d'une seringue dans un tube de Schlenk. Le tube de Schlenk est ensuite équipé d'un condenseur surmonté d'un bulleur. Le système est ensuite chauffé à 120 °C pendant 18 h. Les produits obtenus sont déterminés par RMN et le pourcentage de chaque produit est estimé par la RMN du proton et la chromatographie en phase gazeuse. Le taux de conversion du n-butanol est de l'ordre de 20%. La sélectivité pour le butyrate de butyl est 100%.

### Exemple 2b : synthèse de butyrate de butyl à partir de tributyrate de glyceryl (tributyrine) en absence de solvant :

Le schéma réactionnel de la réaction est le suivant :

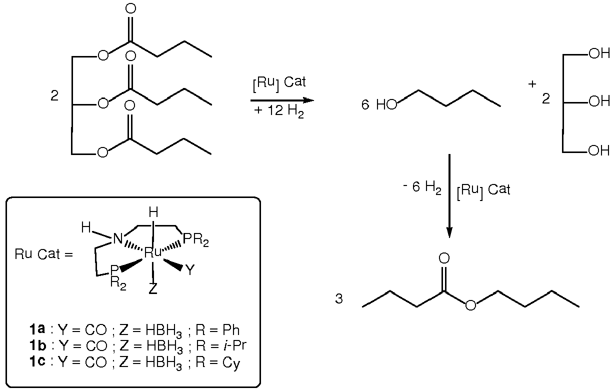

Etape 1 : Dans la boite à gant, 0.1% mol du catalyseur 1a (30 mg ; 51.3 µmol), 5.16 g de tributyrate de glyceryl (1,7 mmol) sont successivement chargés sous atmosphère d'argon dans un autoclave contenant un barreau d'agitation. L'autoclave est ensuite purgé trois fois avec de l'hydrogène puis environ 40 bars d'hydrogène sont introduits. Le système est chauffé à 100 °C à l'aide d'un cordon chauffant électrique et est agité magnétiquement pendant 18 heures. Le mélange réactionnel est analysé par la RMN du proton et la chromatographie en phase gazeuse. Le taux de conversion est de l'ordre 84%. Les rendements pour le n-butanol et le butyrate de butyl sont respectivement 45 et 7%. De plus, la formation des mono et diester glycérides sont également observés avec les pourcentages estimés de l'ordre de 16 et 13%, respectivement.

Etape 2 : Dans la boite à gant, le milieu réactionnel est transféré sous d'argon à l'aide d'une seringue dans un tube de Schlenk. Le tube de Schlenk est ensuite équipé d'un condenseur surmonté d'un bulleur. Le système est ensuite chauffé à 120 °C pendant 18 h. Les produits obtenus sont déterminés par RMN et le pourcentage de chaque produit est estimé par la RMN du proton et la chromatographie en phase gazeuse. Le taux de conversion du n-butanol est de l'ordre de 5%. La sélectivité pour le butyrate de butyl est 100%.

### Exemple 2c : synthèse d'oléate d'oléyle à partir de trioléate de glyceryl (trioléine) :

Le schéma réactionnel de la réaction est le suivant :

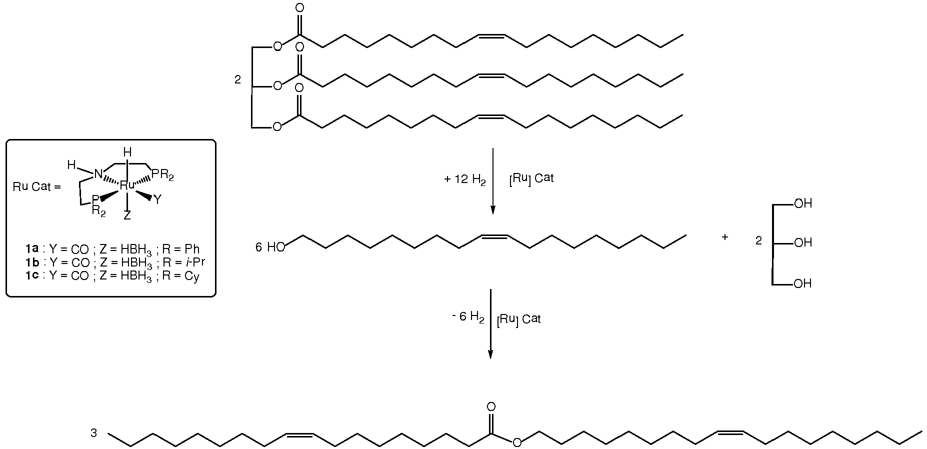

Etape 1 : Dans la boite à gant, 0.33 mol% du catalyseur 1a (3.0 mg ; 5.13 µmol), 0.453 g de tributyrate de glyceryl (0.51 mmol) et toluène (7 mL) sont successivement chargés sous atmosphère d'argon dans un autoclave contenant un barreau d'agitation. L'autoclave est ensuite purgé trois fois avec de l'hydrogène puis environ 40 bars d'hydrogène sont introduits. Le système est chauffé à 100 °C à l'aide d'un cordon chauffant électrique et est agité magnétiquement pendant 18 heures. Le mélange réactionnel est analysé par la RMN du proton et la chromatographie en phase gazeuse. Le taux de conversion est de l'ordre 94%. Les rendements pour l'alcool oléique et l'oléate d'oléyl sont respectivement 87 et 7%. Il est intéressant à noter que les insaturations de la chaine grasse ne sont pas hydrogénées au cours de ce processus.

Etape 2 : Dans la boite à gant, le milieu réactionnel est transféré sous d'argon à l'aide d'une seringue dans un tube de Schlenk. 0.66 mol% du catalyseur 1a a été rajouté (6.0 mg ; 10.26 µmol). Le tube de Schlenk est ensuite équipé d'un condenseur surmonté d'un bulleur. Le système est ensuite chauffé à 120 °C pendant 18 h. Les produits obtenus sont déterminés par RMN et le pourcentage de chaque produit est estimé par la RMN du proton et la chromatographie en phase gazeuse. Il faut noter que les mono et diester glycérides ne se représentent que sous forme de traces.

Les rendements et les taux de conversion obtenus dans les exemples 2a, 2b et 2c au bout de deux étapes sont compilés dans le Tableau III :

**Tableau III**

| **Cat.*** | **Substrat** | **Solvant** | **Rendement Alcool^{b}** | **Rendement Céride^{b}** | **Conversion^{b}** |
|---|---|---|---|---|---|
| 1a, 1 mol% | Tributyrine | Toluène | 72% | 21% | 96% |
| 1a, 0.1 mol% | Tributyrine | Non | 43% | 10% | 84% |
| 1a,^{a} (0.33 mol%) + 0.66 mol% | Trioléine | Toluène | 87% | 7% | 94% |

| | | | | | |
|---|---|---|---|---|---|
| (*) : Rapport catalyseur/fonction ester ; (^{a}) : 0.33 mol% du catalyseur 1a a été utilisé pour la première étape ; 0.66 mol% de 1a a été rajouté dans le milieu réactionnel pour la deuxième étape. (^{b}) : Rendements et conversion estimés par RMN ¹H et CPG. | | | | | |

### Exemple 3 : synthèse du Carbonylchlorohydrido[bis(2-di-isopropylphosphinoethyl)amino]ruthenium(II) (2b) :

Le schéma réactionnel de la réaction est le suivant :

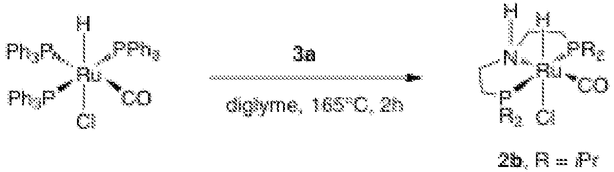

*Synthèse :* Dans un tube de Schlenk une suspension de carbonylchlorohydrido[tris(triphenylphosphine)]ruthenium(II) (Strem Chemicals, 0,999 g ; 1.04 mmol) et de NH(C₂H₄P*i*Pr₂)₂, **3a** (0,357 g ; 1.17 mmol) dans du diglyme (10 mL) est placé dans un bain d'huile préchauffé à 165 °C et laissé sous agitation pendant deux heures pour donner une solution jaune limpide. La solution est laissée 18h à température ambiante pour donner un précipité. 10 mL de pentane sont additionnés et la suspension refroidie à 0 °C pendant 1 heure. Le surnageant est ensuite éliminé et les cristaux sont lavés par de l'éther diéthylique (3 x 5 mL) puis séchés sous pression réduite pour donner le produit désiré sous forme d'une poudre jaune très pâle. Rendement : 64% (0,317 g).
RMN ³¹P-{¹H} (CD₂Cl₂, 121,5 MHz) : δ. 74,7 ppm. RMN ¹H et ³¹P en accord avec les données spectrales de la littérature. Voir : Bertoli, M.; Choualeb, A.; Lough, A. J.; Moore B.; Spasyuk, D.; Gusev D. G. Organometallics, 2011, 30, 3479.

### Exemple 4 : Synthèse de Carbonylchlorohydrido[bis(2-dicyclohexylphosphinoethyl)amino]ruthenium(II) (2c) :

Le schéma réactionnel de la réaction est le suivant :

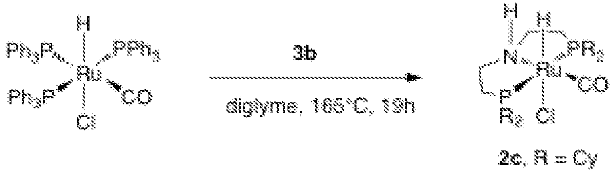

*Synthèse* : Dans un tube de Schlenk une suspension de carbonylchlorohydrido[tris(triphenylphosphine)]ruthenium(II) (Strem Chemicals, 1,000 g ; 1,05 mmol) et NH(C₂H₄PCy₂)₂, **3b** (0,498 g ; 1,07 mmol) dans du diglyme (10 mL) est placé dans un bain d'huile préchauffé à 165 °C et laissé sous agitation pendant 19 heures pour donner une suspension. Le milieu est ensuite refroidi à température ambiante, le surnageant est éliminé et le précipité est lavé par de l'éther diéthylique (3x 5 mL) puis séché sous pression réduite pour donner le produit désiré sous forme d'une poudre jaune très pâle. Rendement : 78 % (0,518 g) RMN³¹P{¹H} (CD₂Cl₂, 121,5 MHz) : δ. 65.6 ppm. RMN ¹H et ³¹P en accord avec les données spectrales de la littérature. Voir : Nielsen, M. ; Alberico, E.; Baumann, W.; Drexler H.-J.; Junge, H.; Gladiali, S.; Beller, M. Nature, 2013, (doi:10.1038/nature11891)

### Exemple 5 : Synthèse du Carbonylhydrido(tetrahydroborato)[bis(2-di-i-propylphosphinoethyl)amino]ruthenium(II) (1b) :

Le schéma réactionnel de la réaction est le suivant :

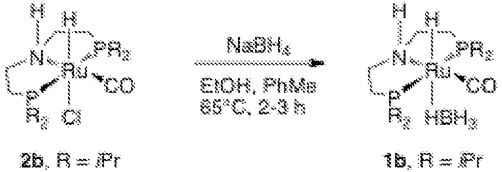

Synthèse : Dans un tube de Schlenk sous flux d'argon, une solution de NaBH4 (5 mg; 0,24 mmol) dans l'éthanol (2 mL) est ajoutée à une suspension de carbonylchlorohydrido[bis(2-di-i-propylphosphinoethyl)amino]ruthenium(II), 2b, (50 mg ; 0,08 mmol) dans le toluène (8mL). Le tube de Schlenk est ensuite fermé hermétiquement, plongé dans un bain d'huile préchauffé à 65 °C et laissé sous agitation pendant 2 h 30 pour donner une solution opalescente. Le solvant est ensuite éliminé par distillation sous pression réduite (température ambiante, 1.10-3 mbar). Le résidu blanc obtenu est extrait par du dichlorométhane (3 x 5 ml) et filtré sur verre fritté. Le filtrat est ensuite concentré sous pression réduite (température ambiante, 1.10-3 mbar) pour donner le produit désiré sous la forme d'une poudre blanche (30 mg ; rendement : 62%).
RMN 1H (CD2Cl2, 300 MHz) : δ. 3,90 (large ; 1H ; NH) ; 3,30-3,12 (m ; 2H) ; 2.56-2.44 (m ; 2H) ; 2.30-2.21 (m ; 2H) ; 1.94 - 1.82 (m ; 2H) ; 1,38 (dd ; JHP=16,2 Hz ; JHH=7,5 Hz ; 6H) ; 1,28-1.14 (m ; 18 H) ; -1,92 - -2,69 (large ; 4H ; RuHBH3) ; -13,53 (t, JHP=17,7 Hz ; 1H; RuH). 31P-{1H} (CD2Cl2, 121,5 MHz) : δ. 77,7 ppm

### Exemple 6 : Synthèse du carbonylhydrido(tetrahydroborato)[bis(2-dicyclohexylphosphinoethyl)amino]ruthenium(II)(1c) :

Le schéma réactionnel de la réaction est le suivant :

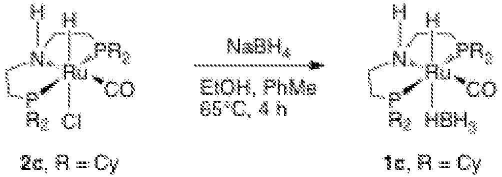

*Synthèse :* Dans un tube de Schlenk sous flux d'argon, une solution de NaBH₄ (48 mg ; 1,37 mmol) dans d'éthanol (12 mL) est ajoutée à une suspension de carbonylchlorohydrido[bis(2- dicyclohexylphosphinoethyl)amino] ruthenium (II), **2c**, (200 mg ; 0,43 mmol) dans le toluène (16 mL). Le tube de Schlenk est ensuite fermé hermétiquement, plongé dans un bain d'huile préchauffé à 65 °C et laissé sous agitation pendant quatre heures pour donner une solution opalescente. Le solvant est ensuite éliminé par distillation sous pression réduite (température ambiante, 1.10⁻³ mbar). Le résidu blanc obtenu est extrait par du dichlorométhane (3 x 5 ml) et filtré sur verre fritté. Le filtrat est ensuite concentré sous pression réduite (température ambiante, 1.10⁻³ mbar) pour donner le produit désiré sous la forme d'une poudre blanche (171 mg ; rendement : 88%).
RMN ¹H (CD₂Cl₂, 300 MHz) : δ. 3,85 (large ; 1H; NH) ; 3,27-3,09 (m ; 2H) ; 2,27-2,10 (m ; 8H) ; 1,96-1,68 (m; 20H) ; 1,61-1,20 (m; 22H) ; -2,19 - -2,50 (*large;* 4H ; Ru*HBH₃*) ;-13,60(t,*J_{HP}*=17, 9Hz ; 1H; Ru*H*). ³¹P-{¹H} (CD₂Cl₂, 121,5 MHz) : δ. 68.9 ppm.

### Exemple 7 : Synthèse du carbonyl(dihydrido)[bis(2-di-isopropylphosphinoethyl)amino]ruthenium(II) (6b) :

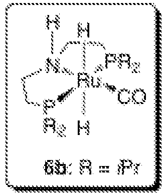

Synthèse : Dans un tube de Schlenk une suspension blanchâtre de carbonylhydridoclhoro[bis(2-di-isopropylphosphinoethyl)amino]ruthénium(II) **2b** (221 mg ; 0,468 mmol) dans du THF (8 ml) est traitée par une solution commerciale de NaHBEt₃ à 1 M dans du toluène (0,45 ml ; 0,450 mmol). Puis, le milieu est laissé sous agitation à température ambiante sous atmosphère d'argon pendant 18 heures pour donner une solution jaune clair opalescente. La solution est ensuite concentrée sous pression réduite pour conduire à un résidu solide jaune lequel est dissout avec du toluène (8 mL). Cette nouvelle solution est filtrée sur verre fritté et concentrée sous pression réduite pour donner une poudre jaune. Rendement 89% (181 mg)
RMN ¹H (C₆D₆, 300 MHz) : δ. 2,20 - 2,04 (m ; 3H) ; 1,92 - 1,83 (m ; 4H) ; 1,66 - 1,54 (m ; 2H) ; 1,36 - 1,27 (m ; 24H) ; 1,01 - 0,91 (m ; 2H) ; -6,18 - -6,46 (m ; 2H ; *RuH₂*).
RMN ³¹P{¹H} (C₆D₆, 121,5 MHz) : δ. 91,1 ppm.

### Exemple 8 : Synthèse du carbonyl(dihydrido)[bis(2-di-cyclohexylphosphinoethyl)amino]ruthénium(II) (6c)

Synthèse : Dans un tube de Schlenk une suspension blanchâtre de carbonylhydridoclhoro[bis(2-di-cyclohexylphosphinoethyl)amino]ruthénium(II) **2c** (63 mg ; 0,1 mmol) dans du THF (2 ml) est traitée par une solution commerciale de NaHBEt₃ à 1 M dans du toluène (0,12 ml ; 0,12 mmol). Puis, le milieu est laissé sous agitation à température ambiante sous atmosphère d'argon pendant 18 heures pour donner une solution jaune clair opalescente. La solution est ensuite concentrée sous pression réduite pour conduire à un résidu solide jaune lequel est dissout avec du toluène (2 mL). Cette nouvelle solution est filtrée sur verre fritté et concentrée sous pression réduite pour donner une poudre jaune. Rendement 80% (48 mg).
RMN ¹H (C₆D₆, 300 MHz) : δ. 2,32 - 2,07 (m ; 6H) ; 1,90 - 1,60 (m ; 31H) ; 1,34 - 1,03 (m ; 16H) ; -6,18 - -6,34 (m ; 2H ; Ru*H₂*)
RMN ³¹P{¹H} (C₆D₆, 121,5 MHz) : δ. 80,4 ppm

## Revendications

1. Procédé de synthèse d'un second ester à partir d'un premier ester qui comprend les étapes suivantes :
- a) la mise en présence d'un premier ester et d'un catalyseur de formule 1 et de dihydrogène pour obtenir un premier alcool et un deuxième alcool;
- b) Séparer ledit deuxième alcool du milieu réactionnel;
- c) faire réagir ledit premier alcool avec le catalyseur de formule 1 de l'étape a) pour obtenir un deuxième ester et du dihydrogène ; et
- d) recycler le dihydrogène obtenu à l'étape c) en l'introduisant à l'étape a) ;
ladite formule 1 étant : 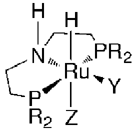
où R sont des groupements, identiques ou différents, choisis dans le groupe constitué par le cyclohexyle, le phényle, l'isopropyle et l'éthyle;
où Y est une phosphine PR'₃, un radical CO ou un atome d'hydrogène, R', identiques ou différents, étant des groupements alkyles en C₁-C₁₂ ou aryle en C₆-C₁₂; et
où Z est un atome d'hydrogène, un groupement HBH₃ ou un atome d'halogène.

2. Procédé selon la revendication 1, **caractérisé en ce que** l'étape c) du procédé est effectuée sans ajout d'un accepteur d'hydrogène.

3. Procédé selon la revendication 1 ou 2, **caractérisé en ce que** la ou les étapes a) et/ou c) du procédé est/sont effectuée(s) sans ajout de solvant.

4. Procédé selon la revendication 3, **caractérisé en ce que** la ou les étapes a) et/ou c) du procédé est/sont effectuée(s) sans ajout de base.

5. Procédé selon l'une quelconque des revendications 1 à 4, **caractérisé en ce que** Z n'est pas un atome d'halogène.

6. Procédé selon l'une quelconque des revendications 1 à 5, **caractérisée en ce que** le groupement R' est un groupement méthyle, éthyle, isopropyle ou phényle.

7. Procédé selon l'une quelconque des revendications 1 à 6, **caractérisée en ce que** R est un groupement cyclohexyle ou isopropyle.

8. Procédé selon l'une quelconque des revendications 1 à 7, **caractérisée en ce que** ledit premier ester présente la formule suivante : 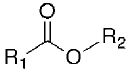 où R₁ est un groupement alkyle de C₂ à C₃₂, et R₂ est un groupement alkyle de C₁ à C₆.

9. Procédé selon l'une quelconque des revendications 1 à 7, **caractérisée en ce que** ledit premier ester présente la formule suivante : 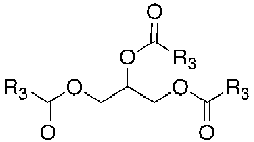 où R₃ sont des groupements alkyles de C₂ à C₃₂, identiques ou différents.

10. Procédé selon l'une quelconque des revendications 1 à 9, **caractérisé en ce que** la charge dudit catalyseur de formule 1 utilisé à l'étape b) est choisie dans une gamme allant de 500 ppm à 1 ppm, avantageusement de 200 ppm à 20 ppm, plus avantageusement de 60 ppm à 40 ppm.

11. Procédé selon l'une quelconque des revendications 1 à 10, **caractérisé en ce que** l'étape a) est réalisée dans des conditions réactionnelles comprenant :
i) une température allant de 5°C à 150°C, avantageusement de 75°C à 125°C, plus avantageusement de 100°C ;
ii) une pression de dihydrogène allant de 1 bar à 50 bar, avantageusement de 10 à 30 bar, plus avantageusement de 20 bar.

12. Procédé selon l'une quelconque des revendications 1 à 11, **caractérisé en ce que** l'étape c) est réalisée dans des conditions réactionnelles comprenant :
i) une température allant de 5°C à 200°C, avantageusement de 100°C à 150°C, plus avantageusement de 130°C ;
ii) une pression permettant le dégagement concomitant d'hydrogène.

13. Procédé selon la revendication 12, **caractérisé en ce que** ladite pression permettant le dégagement d'hydrogène est une pression égale ou inférieure à la pression atmosphérique.

14. Utilisation d'un catalyseur de formule 1 dans un procédé tel que décrit à l'une quelconque des revendications 1 à 13.

## Patentansprüche

1. Verfahren zur Synthese eines zweiten Esters aus einem ersten Ester, das die folgenden Schritte umfasst:
- a) Inberührungbringen von einem ersten Ester und einem Katalysator der Formel 1 und Diwasserstoff zum Erhalt eines ersten Alkohols und eines zweiten Alkohols;
- b) Abtrennen des zweiten Alkohols vom Reaktionsmedium;
- c) Umsetzen des genannten ersten Alkohols mit dem Katalysator der Formel 1 aus Schritt a) zum Erhalt eines zweiten Esters und Diwasserstoff; und
- d) Rezyklieren des in Schritt c) erhaltenen Diwasserstoffs durch Eintragen in Schritt a);
wobei es sich bei der Formel 1 um: 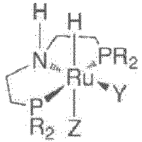 handelt,
wobei R für gleiche oder verschiedene Gruppen, die aus der Gruppe bestehend aus Cyclohexyl, Phenyl, Isopropyl und Ethyl ausgewählt sind, stehen;
wobei Y für ein Phosphin PR'₃, einen CO-Rest oder ein Wasserstoffatom steht, wobei R' gleich oder verschieden sind und für C₁-C₁₂-Alkyl- oder C₆-C₁₂-Arylgruppen stehen; und
wobei Z für ein Wasserstoffatom, eine HBH₃-Gruppe oder ein Halogenatom steht.

2. Verfahren nach Anspruch 1, **dadurch gekennzeichnet, dass** man den Schritt c) des Verfahrens ohne Zusatz eines Wasserstoffakzeptors durchführt.

3. Verfahren nach Anspruch 1 oder 2, **dadurch gekennzeichnet, dass** man den Schritt oder die Schritte a) und/oder c) des Verfahrens ohne Zusatz von Lösungsmittel durchführt.

4. Verfahren nach Anspruch 3, **dadurch gekennzeichnet, dass** man den Schritt oder die Schritte a) und/oder c) des Verfahrens ohne Zusatz von Base durchführt.

5. Verfahren nach einem der Ansprüche 1 bis 4, **dadurch gekennzeichnet, dass** Z nicht für ein Halogenatom steht.

6. Verfahren nach einem der Ansprüche 1 bis 5, **dadurch gekennzeichnet, dass** die Gruppe R' für eine Methyl-, Ethyl-, Isopropyl- oder Phenylgruppe steht.

7. Verfahren nach einem der Ansprüche 1 bis 6, **dadurch gekennzeichnet, dass** R für eine Cyclohexyl- oder Isopropylgruppe steht.

8. Verfahren nach einem der Ansprüche 1 bis 7, **dadurch gekennzeichnet, dass** der erste Ester die folgende Formel aufweist: 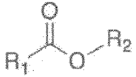 wobei R₁ für eine C₂- bis C₃₂-Alkylgruppe steht und R₂ für eine C₁- bis C₆-Alkylgruppe steht.

9. Verfahren nach einem der Ansprüche 1 bis 7, **dadurch gekennzeichnet, dass** der genannte erste Ester die folgende Formel aufweist: 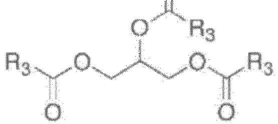 wobei R₃ für gleiche oder verschiedene C₂- bis C₃₂-Alkylgruppen stehen.

10. Verfahren nach einem der Ansprüche 1 bis 9, **dadurch gekennzeichnet, dass** man die Beladung des in Schritt b) verwendeten Katalysators der Formel 1 aus einem Bereich von 500 ppm bis 1 ppm, vorteilhafterweise von 200 ppm bis 20 ppm, noch vorteilhafter von 60 ppm bis 40 ppm wählt.

11. Verfahren nach einem der Ansprüche 1 bis 10, **dadurch gekennzeichnet, dass** man den Schritt a) unter Reaktionsbedingungen durchführt, die Folgendes umfassen:
i) eine Temperatur im Bereich von 5 °C bis 150 °C, vorteilhafterweise von 75 °C bis 125 °C, noch vorteilhafter von 100 °C;
ii) einen Diwasserstoffdruck im Bereich von 1 bar bis 50 bar, vorteilhafterweise von 10 bis 30 bar und noch vorteilhafter von 20 bar.

12. Verfahren nach einem der Ansprüche 1 bis 11, **dadurch gekennzeichnet, dass** man den Schritt c) unter Reaktionsbedingungen durchführt, die Folgendes umfassen:
i) eine Temperatur im Bereich von 5 °C bis 200 °C, vorteilhafterweise von 100 °C bis 150 °C, noch vorteilhafter von 130 °C;
ii) einen Druck, der die gleichzeitige Freisetzung von Wasserstoff erlaubt.

13. Verfahren nach Anspruch 12, **dadurch gekennzeichnet, dass** es sich bei dem Druck, der die gleichzeitige Freisetzung von Wasserstoff erlaubt, um einen Druck handelt, der gleich oder kleiner als Normaldruck ist.

14. Verwendung eines Katalysators der Formel 1 beim Verfahren gemäß einem der Ansprüche 1 bis 13.

## Claims

1. Method for synthesizing a second ester from a first ester which comprises the following steps:
- a) bringing a first ester and a catalyst of formula 1 and dihydrogen into contact in order to obtain a first alcohol and a second alcohol;
- b) separating said second alcohol from the reaction medium;
- c) reacting said first alcohol with the catalyst of formula 1 from step a) in order to obtain a second ester and dihydrogen; and
- d) recycling the dihydrogen obtained in step c) by introducing it into step a);
said formula 1 being: 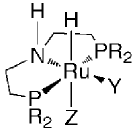
where R are groups, which may be identical or different, selected from the group consisting of cyclohexyl, phenyl, isopropyl and ethyl;
where Y is a PR'₃ phosphine, a CO radical or a hydrogen atom, R', which are identical or different, being C₁-C₁₂ alkyl groups or C₆-C₁₂ aryl groups; and
where Z is a hydrogen atom, a HBH₃ group or a halogen atom.

2. Method according to Claim 1, **characterized in that** step c) of the method is carried out without addition of a hydrogen acceptor.

3. Method according to Claim 1 or 2, **characterized in that** the step(s) a) and/or c) of the method is/are carried out without addition of solvent.

4. Method according to Claim 3, **characterized in that** the step(s) a) and/or c) of the method is/are carried out without addition of base.

5. Method according to any one of Claims 1 to 4, **characterized in that** Z is not a halogen atom.

6. Method according to any one of Claims 1 to 5, **characterized in that** the R' group is a methyl, ethyl, isopropyl or phenyl group.

7. Method according to any one of Claims 1 to 6, **characterized in that** R is a cyclohexyl or isopropyl group.

8. Method according to any one of Claims 1 to 7, **characterized in that** said first ester has the following formula: 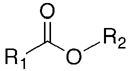 where R₁ is a C₂ to C₃₂ alkyl group, and R₂ is a C₁ to C₆ alkyl group.

9. Method according to any one of Claims 1 to 7, **characterized in that** said first ester has the following formula: 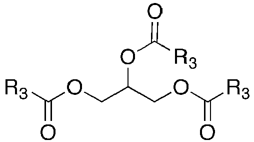 where R₃ are identical or different C₂ to C₃₂ alkyl groups.

10. Method according to any one of Claims 1 to 9, **characterized in that** the loading of said catalyst of formula 1 used in step b) is selected from a range extending from 500 ppm to 1 ppm, advantageously from 200 ppm to 20 ppm, more advantageously from 60 ppm to 40 ppm.

11. Method according to any one of Claims 1 to 10, **characterized in that** step a) is carried out under reaction conditions comprising:
i) a temperature ranging from 5°C to 150°C, advantageously from 75°C to 125°C, more advantageously of 100°C;
ii) a pressure of dihydrogen ranging from 1 bar to 50 bar, advantageously from 10 to 30 bar, more advantageously of 20 bar.

12. Method according to any one of Claims 1 to 11, **characterized in that** step c) is carried out under reaction conditions comprising:
i) a temperature ranging from 5°C to 200°C, advantageously from 100°C to 150°C, more advantageously of 130°C;
ii) a pressure that allows the concomitant release of hydrogen.

13. Method according to Claim 12, **characterized in that** said pressure that allows the release of hydrogen is a pressure equal to or below atmospheric pressure.

14. Use of a catalyst of formula 1 in a method as described in any one of Claims 1 to 13.
